# EUROPEAN PATENT SPECIFICATION

(11) **EP 1 933 923 B1**
(45) Date of publication and mention of the grant of the patent: **04.02.2009**
(21) Application number: 06779255.6
(22) Date of filing: 01.09.2006
(51) Int. Cl.: A61M 25/02

(54) **INFUSION LINE STABILISER**
STABILISATOR FÜR EINE INFUSIONSLEITUNG
STABILISATEUR DE CONDUIT DE PERFUSION

(30) Priority: 16.09.2005 GB 0518922
(43) Date of publication of application: 25.06.2008
(73) Proprietor: Shams, Iden, 35 Norton Road Uxbridge London UB8 2PT (GB)
(72) Inventor: Shams, Iden, 35 Norton Road Uxbridge London UB8 2PT (GB)
(74) Representative: Dixon, Philip Matthew
(86) International application number: PCT/GB2006/003234
(87) International publication number: WO 2007/031707

(56) References cited:
- WO-A-00/24313
- WO-A-03/082396
- WO-A2-92/22041

## Description

### Field of the Invention

The invention relates to devices for stabilising an intravenous infusion catheter by holding it on a fixed position on a patient's body such a device is disclosed in the WO 03082396.

### Background and Prior Art known to the Applicant

Devices for stabilisation of catheters used for intravenous therapy are known in themselves. US patent 5,346,478 (Schneider) discloses a retention device for drainage tubes and the like, having a pad for adhesive attachment to a patient's skin and having a strap to be looped and tightened around a drainage tube for immobilising the tube.

US patent 4,311,137 (Gerard) discloses a fluid administration device with a catheter holder having a cannula and a fluid administration side port. To aid positioning and stabilisation of the infusion device, a pair of diametrically opposed flat wings is provided which may be adhesively taped to the skin of the patient at the venipuncture site to maintain the device stationary during infusion.

US patent 2002/012031 (Douglas) discloses a subcutaneous injection set having an internal Y-shaped flow channel connected to an injection needle.

US patent 6,626,884 (Dillon) discloses apparatus for use in blood connection (rather than in intravenous therapy) comprising a multiport rotary valve and a wrist strap. The document teaches that the valve can be attached to the strap by a hook and loop fastening system (such as sold under the registered trade mark Velcro) or the like, i.e. using a reversibly releasable fixing. The inventor explains that 'the immobilisation of the blood line in this way is an added advantage for the comfort of the donor and may avoid the need for the use of a conventional adhesive strip for this purpose'.

US patent 6,086,564 discloses a wrist-mounted intravenous administration set comprising a synthetic resin infusion body supported by flexible adhesive mount allowing the assembly to be secured on a patient's body at a desired infusion site. The multiple-port infusion body is detachably supported on a mount in the form of a flexible strap designed to wrap partially around a patient's extremity, most commonly the wrist. The inner surface of the strap has pressure sensitive skin adhesive allowing the strap to be releasably attached to the patient's body.

US patent 2,449,882 (Daniels) discloses a holder for intravenous apparatus for positioning and stabilising tubing for intravenous injections. The device comprises a limb-encircling strap fixed with a conventional button and buttonhole arrangement and a clip to releasably hold an intravenous infusion tube.

US patent 5,263,943 (Vanderbrook) discloses a valved intravenous needle assembly comprising a wrist strap and a cradle support to hold an intravenous needle by the use of a releasable hook and loop fastener.

US patent 4,846,807 (Safadago) discloses an intravenous tube anchor and shield. The device has as its primary object the shielding of the area of the body into which an IV or other needle for introducing therapeutic liquid is inserted and for anchoring the IV tube adjacent to the point of insertion. The device has means to anchor an IV tube and is secured to the body of a patient by straps, fastened with hook and loop fastenings.

US patent 4,666,434 (Kaufman) discloses a catheter locating device having a rotatable `saddle' mounted on an anchoring bracelet in which the saddle includes an aperture adapted to receive a catheter. The anchoring bracelet fixes to eg. the wrist of a user with a clasp or, again, a hook and loop closure.

US patent 4,316,461 (Marais) discloses an intravenous vascular stabiliser. The device comprises a base plate with a longitudinal aperture adapted to sit either side of a vein of a patient when the design is placed against, eg. a forearm. The device has a number of clips to hold the tubes of a catheter in place and straps fastened by a button to secure the device in place.

US patent 3,939,832 (Miller) discloses a liquid flow regulator and monitor for an infusion system. The apparatus has a liquid control valve and flow meter positioned on a patient adjacent the point of entry the fluid into the body. The apparatus is strapped to the wrist of a patient by the use of a button hole mechanism.

US patent 3,782,382 (Naftulin) discloses fluid or blood administration equipment including a plastic manifold which is connectable to a wrist of a user by a strap fastened, again, with a hook and loop closure.

US patent 2,261,213 (Bierman) discloses a syringe adaptor for use in intravenous infusions. In use, the adaptor is attached to the arm of a patient by means of adhesive tape.

In this field of technology, it can be seen that these developments, spanning over 60 years, have all attempted to provide solutions to the problem of stabilisation of intravenous infusion apparatus. The applicant recognises the key criteria of isolating the site of infusion from disturbance of the 'upstream' infusion apparatus and the prevention of infection. The present invention addresses these problems.

### Summary of the Invention

The invention provides an infusion line stabiliser comprising: a multiport body providing a fluid exchange route between a first port and at least a second port; and a band, attached or attachable to the multiport body and configured to be able to fit, in use, onto a user's limb to hold the body near an intravenous therapy location wherein the band is a strap, sized to encircle the user's limb; characterised by the following combination of features: that the strap and body incorporates a one-way-only locking means to secure the band, non-releasably, to the body after encircling a user's limb, thereby requiring the band, or the multiport body, to be severed to release the stabiliser from the user's limb.

The one-way locking means is a reversal of the teaching of the known art where similar devices are provided with reversibly attachable bands, most commonly using hook and loop closures, but sometimes with button - buttonhole arrangements. The present, irreversibly attachable, single use device has a number of distinct advantages: patients requiring stabilization of intravenous therapy apparatus might be mentally confused, distressed and anxious; the locking feature ensures that they cannot inadvertently remove the device, leaving the site of venipuncture unprotected. The same benefits also accrue when the device is to be used with children, who have a tendency to play with devices, especially when bored, and who might not understand the importance of leaving the stabiliser in place. When used in a veterinary context, where patient-clinician communication is problematic, the benefit is very great indeed. Secondly, because the device must be effectively "destroyed" by cutting the band or breaking the body to remove it from a patient, it prevents accidental re-use of the device, which could lead to cross-infection from one patient to another.

In order to prevent accidental removal, it is preferable that the band is substantially inelastic. In any aspect of the invention, it is particularly advantageous if the multiport body has more than two ports. Whilst a two-port device has benefits as a secure stabilization device, and effectively acts as an inline connector, when more ports are provided, it allows a supervising clinician to administer more than one medication through the same device. For example, a patient might have an intravenous drip administered continuously through one port, and have periodic medication administered using a second port.

In any aspect of the device, it is particularly advantageous if at least one point of attachment of the band to the multiport body is configured to project from the multiport body's limb-contacting surface so as to lift the band from the encircled limb as the band approaches the point of attachment. This provides a number of unexpected benefits: firstly, it makes it considerably more easy to connect a free end of the strap to the multiport body as the point of attachment is held away from the patient's limb. Secondly, if the multiport body is made of a substantially rigid material (which would usually be the case) the extra width of the multiport body serves to resist rotation movement of the device on the patient's limb. This is especially so when the device is fitted to the wrist, which has an almost "rounded rectangular" cross-section. The combination of a narrow multiport body and a flexible strap can rotate on the wrist, whereas a wide, i.e. extended with such a projection, body prevents this movement.

Also in any aspect of the invention, it is preferable that the multiport body comprises a multi-way valve, and wherein the valve has a rotatable top with a number of projecting arms to aid a user in rotating it; characterised in that the arm or arms are arranged to indicate the available flow paths in the valve body by aligning each with a respective one of the visible lead-ins or exits from each port.

In any aspect of the invention, it is preferable that the locking means comprises a peg and one-way clasp, configured to engage with a hole in the band, and more preferably that the peg is angled or so-shaped as to hold the band in position by engagement with the hole therein, prior to locking with the clasp.

In a particularly advantageous aspect, the clasp, when locked, fits substantially flush with, or recessed below, the adjacent surfaces of the locking means so as to substantially resist any subsequent attempt to prise it open. This feature adds to the device's security, especially when used with patients who might attempt to remove it when in a confused or distressed state.

Considering the prior art known to the applicant, it can be seen that no known device comprises this unique combination of features to provide the required mechanical and biological security, and in particular:
Schneider, Gerard and Douglas do not have the feature of a limb-encircling band to safely secure the device to a patient. Dillon teaches the use of a releasable hook and loop closure to fix the device to a user's limb and further that the valve is also attached to the strap by Velcro or the like. McLaughlin does not use a limb-encircling band, but teaches that the 'open bracelet' strap must be coated with pressure sensitive skin adhesive to allow the strap to be releasably detached to the patient's body. The device of Daniels does not have a multiport body, and the attachment strap is fastened by means of a releasable button-button hole arrangement. The device of Vanderbrook again teaches fastening an intravenous needle assembly to a wrist strap with a releasable hook and loop fastener system. The device of Safadago again does not have a multiport body and the teaching is to provide a fixing mechanism to a user's body in the form of a hook and loop closure. The device of Kaufman again does not provide a multiport body but merely a positioning channel for a separate needle and catheter arrangement. Furthermore, the document teaches fixing the device to a user's wrist by use of hook and loop fasteners.

The device of Marais again does not disclose a multiport body, and the device is attached to a user's wrist with a button-button hole arrangement. The device of Miller again teaches an attachment mechanism comprising a button-button hole assembly. The device of Naftulin again repeats the teaching of securing the device to a patient's wrist by means of a hook and loop closure. Finally, the device of Bierman teaches that the device is to be fixed to a user's arm by the use of adhesive tape.

### Brief Desciption of the Drawings

The invention will be described with reference to the accompanying drawings, in which:
Figure 1 is a perspective view of a stabiliser according to the present invention;
Figure2 is a cross-section of a stabilizer fitted to a patient's wrist, and illustrating the projecting side portion;
Figures 3 and 4 show details of peg design and configuration to provide for temporary strap location;
Figure 5 illustrates a detailed design of locking mechanism, in cross-section;
Figure 6 is a plan view of the locking mechanism of figure 5;
Figure 7 is a cross-sectional view of showing interaction of a hinged locking mechanism with a strap.
Figure 8 illustrates a preferred arrangement of valve top.

### Description of the Preferred Embodiments

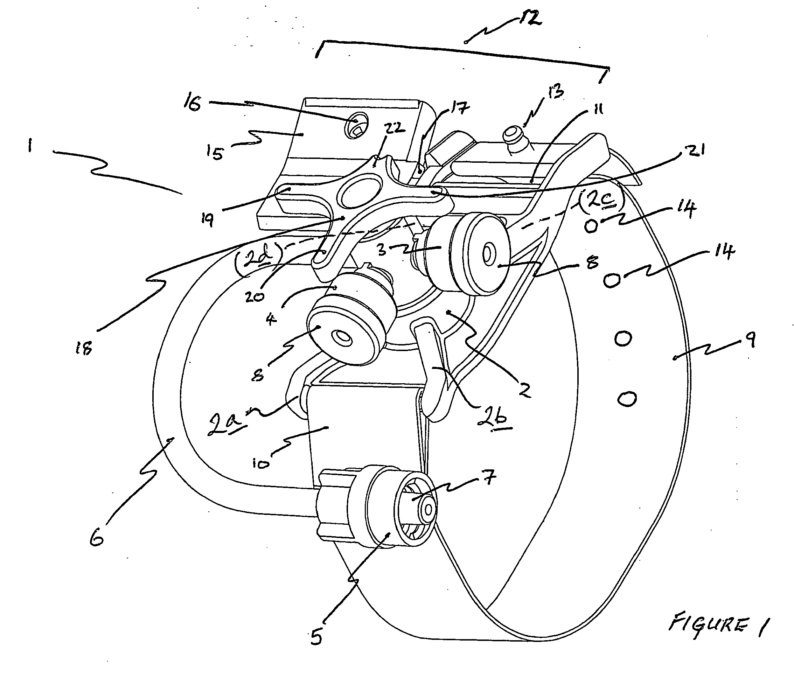

Figure 1 is a perspective view of an infusion line stabiliser generally indicated by 1, according to the present invention. The device has a multiport body 2 having in this instance three ports, 3, 4, 5. One of these ports 5 is connected to the multiport body 2 by means of a flexible tube 6. The port 5 has Luer connection 7 enabling it to be conveniently attached to a needle for insertion into a patient, eg. intravenously or subcutaneously. Ports 3 and 4 are provided with sealing caps 8 to maintain sterility of the device after removing from its sterile packaging.

The device 1 is attachable to a patient's limb (eg. a wrist) by means of a flexible transparent plastics band 9 which is substantially inextensible. The band is attached at one end 10 by looping the band 9 through a slot in the multiport body 2 and heat-sealing it to itself to create a permanent connection.

At the opposite end of the multiport body 2 is a second slot 11 through which the band 9 may be passed for engagement with the locking mechanism, generally indicated by 12. The locking mechanism 12 (described in more detail below) has a projecting peg 13 for engagement with one of a series of holes 14 along the length of the band 9. The provision of a series of spaced holes allows the device to be used on limbs of differing diameters. The locking means 12 also comprises a hinged portion 15 having a one-way clasp 16. The hinged portion 15 is joined to the multiport body by means of a hinge 17, arranged so that, when closed, the clasp engages, non-releasably, with the peg 13.

In this embodiment, the multiport body comprises a multiway valve. The valve has a rotatable top 18 with projecting arms 19, 20, 21, 22 to aid a user in rotating it. The arms are arranged to indicate available flow paths in the valve body by aligning each with a respective one of the visible lead-ins or exits from each port. It can be seen in this embodiment that one of the arms 22 is shorter, and differently shaped to the others (19, 20, 21) indicating that a flow path is available in this configuration between all three ports, 3, 4, 5. Rotation of the valve top by a quarter turn in a clockwise direction to align arm 22 with visible port 3 would indicate a flow path only between port 5 and port 4. Such an arrangement of arms provides not only a quick and visible indication of the status of the valve setting to avoid mis-alignment, but the tactile feedback to an operator enables rapid adjustment of the configuration with minimisation of any chance of error.

The multiport body 2 is preferably made of a rigid plastics material, and can be formed eg by injection moulding. Rigidity is enhanced by ribs 2a, 2b, 2c, 2d which span the body'f flat base plate 2e (and are formed integrally with it) to form an "X" in plan.

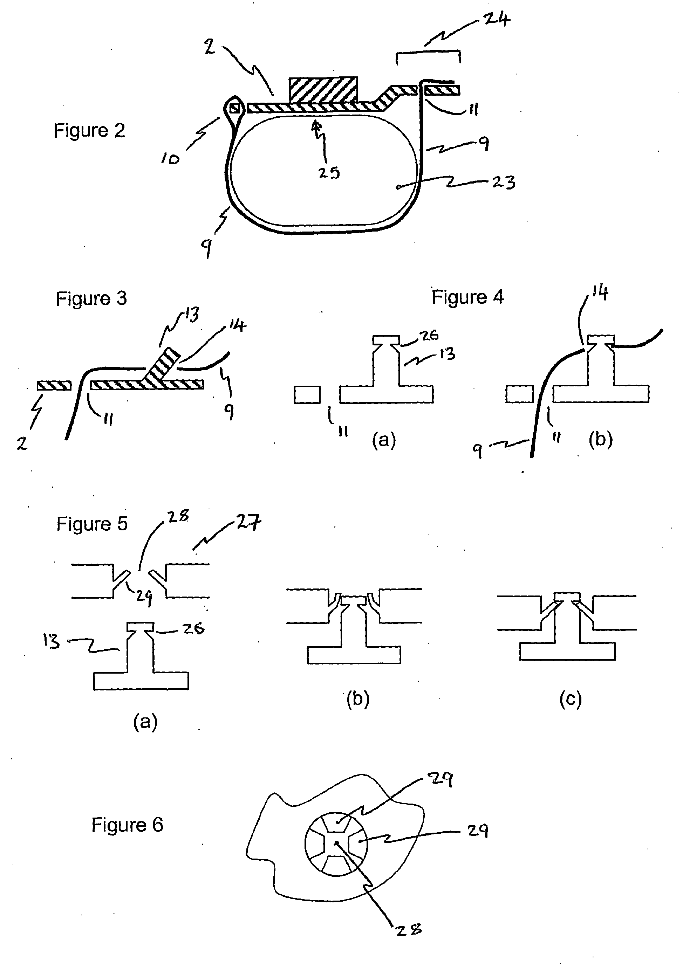

Figure 2 is a schematic cross section through an infusion line stabiliser, located on the wrist 23 of a user. In this instance, the multiport body has a portion 24 configured to project from the multiport body's limb - contacting surface 25 so as to lift the band 9 from the encircled limb 23 as the band approaches the point of attachment. For clarity, the locking mechanism is omitted from this diagram, and the valve body is shown only schematically.

Figure 3 is a detail of a particularly preferred form of attachment means. The band 9 passes through a slot 11 in the multiport body 2 which has a peg 13 inclined at an angle away from the slot 11 so that when a user engages one of the holes 14 in the band 9 over the peg 13 the band 9 is held in position by the engagement, prior to locking with the locking means. By provision of this feature, a supervising clinician does not have to hold the band in place whilst operating the operation may therefore be carried out quickly, with minimum disturbance to the patient.

Figure 4 illustrates an alternative embodiment where the peg 13 is shaped to hold the band 9 in position. The peg 13, of generally circular cross section, is provided with a circumferential groove 26 adjacent its top end. (Figure 4A) The band 9 may be threaded through an adjacent slot 11 and one of the holes 14 engaged with the peg 13. The edge of the hole 14 can then engage with the circumferential slot 26 to prevent the band 9 slipping back through the slot 11. This particular design of peg 13 has special advantages because it can also serve as part of the one-way closure mechanism.

Figure 5 (A-C) illustrates the operation of a suitable peg and one-way clasp mechanism. The peg 13, mounted to the multiport body, has an angled groove 26 towards its top end. The clasp 27 (also illustrated in plan view in figure 6) has a hole 28 around which is disposed a number of upwardly projecting tabs 29 of a resiliently deformable material. As the clasp 27 is brought into engagement with the peg 13, as illustrated in figure 5B, the tabs 29 deform to slide past the upper portion of the peg 13. As the clasp 27 is closed further, the tabs 29 spring back to engage with the circumferential groove 26, thereby locking the two elements together non-releasably.

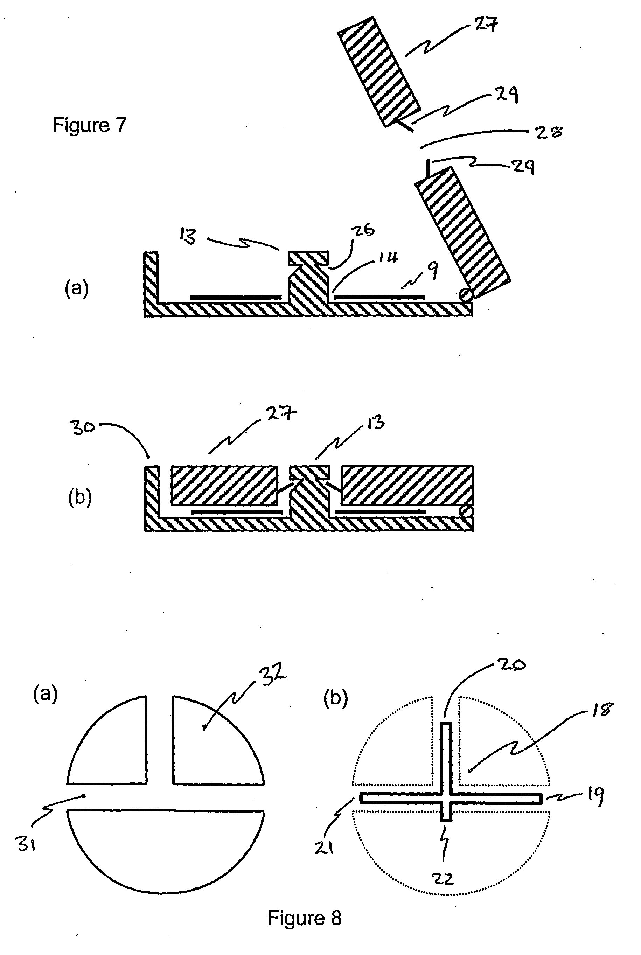

Figure 7 illustrates a cross section through such a locking mechanism showing the clasp 27 hingeably mounted to the peg element. The band 9 is illustrated, again in cross section, with one of its holes 14 in engagement with the peg 13. When the clasp 27 is hinged downwardly to engage, non-releasably, with the peg 13 (as illustrated in figure 7B) the configuration of the clasp and peg element are such that, when locked, the clasp 27 fits substantially flush with, all recess below, the adjacent surfaces of the locking means, so as to substantially resist any subsequent attempt to prise it open.

Figure 8A illustrates, schematically, the flow path 31 within the valve body 32 of a multi-way valve. Figure 8B illustrates the configuration of a rotatable valve top, generally indicated by 18, shown in relation to the flow path (the dotted figure). Valve top 18 has a number of projecting arms (19-22), arranged to indicate the available flow paths within the valve body 32. In this example, one of the arms 22 is shorter than the others to indicate no available flow path in that direction.

As the device is primarily for use in a medical context, it is envisaged that it will be sealed within a protective cover and sterilized after manufacture.

## Claims

1. An infusion line stabiliser (1) comprising:
a multiport body (2) providing a fluid exchange route between a first port (3) and at least a second port (4); and
a band (9), attached or attachable to the multiport body (2) and configured to be able to fit, in use, onto a user's limb to hold the body near an intravenous therapy location wherein the band is a strap, sized to encircle the user's limb;
**characterised by** the following combination of features:
that the strap and body incorporates a one-way-only locking means (12) to secure the band (9), non-releasably, to the body after encircling a user's limb, thereby requiring the band (9), or the multiport bod (2), to be severed to release the stabiliser (1) from the user's limb.

2. An infusion line stabiliser according to claim 1 wherein the band (9) is substantially inelastic.

3. An infusion line stabiliser according to any preceding claim wherein at least one point of attachment of the band (9) to the multiport body (2) is configured to project from the multiport body's limb-contacting surface so as to lift the band from the encircled limb as the band approaches the point of attachment.

4. An infusion line stabiliser according to any preceding claim and wherein the multiport body comprises a multi-way valve, and wherein the valve has a rotatable top (18) with a number of projecting arms (19, 20, 21, 22) to aid a user in rotating it; **characterised in that** the arm or arms are arranged to indicate the available flow paths in the valve body by aligning each with a respective one of the visible lead-ins or exits from each port.

5. An infusion line stabiliser according to any preceding claim wherein the locking means (12) comprises a peg (13) and one-way clasp (16), configured to engage with a hole (14) in the band.

6. An infusion line stabiliser according to claim 5 wherein the peg (13) is angled or so-shaped as to hold the band (9) in position by engagement with the hole (14) therein, prior to locking with the clasp (16).

7. The invention of claim 5 or claim 6 and wherein the clasp (16), when locked, fits substantially flush with, or recessed below, the adjacent surfaces of the locking means (12) so as to substantially resist any subsequent attempt to prise it open.

## Patentansprüche

1. Infusionsleitungs-Stabilisator (1), umfassend:
ein Gehäuse mit mehreren Anschlüssen (2), das einen Flüssigkeitsaustauschweg zwischen einem ersten Anschluss (3) und mindestens einem zweiten Anschluss (4) bereitstellt; und
ein Band (9), das an dem Gehäuse mit mehreren Ausgängen (2) angebracht ist oder angebracht werden kann und dafür ausgelegt ist, bei Verwendung an eine Gliedmaße eines Anwenders zu passen, um das Gehäuse in der Nähe eines intravenösen Therapiezugangs zu fixieren;
wobei das Band ein Gurt ist, der lang genug ist, um die Gliedmaße des Anwenders zu umschließen;
**gekennzeichnet durch** folgende Kombination von Merkmalen:
dass Gurt und Gehäuse ein Einweg-Arretierungsmittel (12) aufweisen, um das Band (9) unlösbar an dem Gehäuse zu befestigen, nachdem es die Gliedmaße eines Anwenders umschließt, sodass das Band (9) oder das Gehäuse mit mehreren Anschlüssen (2) abgetrennt werden muss, um den Stabilisator (1) von der Gliedmaße des Anwenders zu entfernen.

2. Infusionsleitungs-Stabilisator nach Anspruch 1, wobei das Band (9) im Wesentlichen unelastisch ist.

3. Infusionsleitungs-Stabilisator nach einem der vorstehenden Ansprüche, wobei mindestens ein Befestigungspunkt des Bandes (9) an dem Gehäuse mit mehreren Anschlüssen (2) dafür vorgesehen ist, von der die Gliedmaße berührenden Seite des Gehäuses mit mehreren Anschlüssen aus vorzustehen, um das Band von der umschlossenen Gliedmaße abzuheben, wenn das Band sich dem Befestigungspunkt nähert.

4. Infusionsleitungs-Stabilisator nach einem der vorstehenden Ansprüche, wobei das Gehäuse mit mehreren Anschlüssen ein Mehrwegeventil umfasst und wobei das Ventil einen drehbaren Oberteil (18) mit einer Reihe daraus hervorragender Arme (19, 20, 21, 22) besitzt, um dem Anwender das Drehen zu erleichtern; **dadurch gekennzeichnet, dass** der Arm oder die Arme so angeordnet sind, dass sie die verfügbaren Fließwege in dem Ventilgehäuse anzeigen, indem sie jeweils mit einem entsprechenden der sichtbaren Ein- oder Ausgänge von jedem Anschluss gefluchtet sind.

5. Infusionsleitungs-Stabilisator nach einem der vorstehenden Ansprüche, wobei das Arretierungsmittel (12) einen Stift (13) und eine Einweg-Schließe (16) umfasst, die dafür ausgelegt sind, in ein Loch (14) in dem Band einzugreifen.

6. Infusionsleitungs-Stabilisator nach Anspruch 5, wobei der Stift (13) angewinkelt oder derart geformt ist, dass er das Band (9) durch Eingriff in das Loch (14) darin in Position hält, bevor es mit der Schließe (16) arretiert wird.

7. Erfindung nach Anspruch 5 oder Anspruch 6, wobei die Schließe (16), wenn sie arretiert ist, im Wesentlichen bündig mit den angrenzenden Flächen des Arretierungsmittels (12) oder vertieft unterhalb davon liegt, um im Wesentlichen jedem folgenden Versuch, sie zu öffnen, entgegenzuwirken.

## Revendications

1. Un stabilisateur de tube de perfusion (1) comportant :
un corps à orifices multiples (2) fournissant un trajet d'échange de fluide entre un premier orifice (3) et au moins un deuxième orifice (4) ; et
une bande (9), attachée ou attachable au corps à orifices multiples (2) et configurée pour pouvoir s'adapter, lors de l'utilisation, sur un membre d'un utilisateur pour maintenir le corps près d'un emplacement de thérapie intraveineuse ;
dans lequel la bande est un bandage, calibré pour entourer le membre de l'utilisateur ;
**caractérisé par** la combinaison suivante de caractéristiques :
que le bandage et le corps incorporent un moyen de verrouillage seulement unidirectionnel (12) pour assujettir la bande (9), de façon à ce qu'elle ne puisse pas être libérée, au corps après avoir entouré un membre d'un utilisateur, ce qui nécessite ainsi que la bande (9), ou le corps à orifices multiples (2), soit sectionné(e) pour libérer le stabilisateur (1) du membre de l'utilisateur.

2. Un stabilisateur de tube de perfusion selon la revendication 1, dans lequel la bande (9) est essentiellement inélastique.

3. Un stabilisateur de tube de perfusion selon n'importe quelle revendication précédente, dans lequel au moins un point d'attache de la bande (9) au corps à orifices multiples (2) est configuré pour faire saillie à partir de la surface de contact avec le membre du corps à orifices multiples de façon à soulever la bande du membre entouré à mesure que la bande approche du point d'attache.

4. Un stabilisateur de tube de perfusion selon n'importe quelle revendication précédente et dans lequel le corps à orifices multiples comporte une valve multivoies, et dans lequel la valve a un dessus pouvant tourner (18) avec plusieurs bras faisant saillie (19, 20, 21, 22) pour aider un utilisateur à le tourner ; **caractérisé en ce que** le bras ou les bras sont agencés pour indiquer les voies d'écoulement disponibles dans le corps de valve en alignant chacun avec une entrée ou une sortie visible respective parmi les entrées ou les sorties visibles de chaque orifice.

5. Un stabilisateur de tube de perfusion selon n'importe quelle revendication précédente, dans lequel le moyen de verrouillage (12) comporte une cheville (13) et un crochet unidirectionnel (16), configurés pour se mettre en prise avec un trou (14) dans la bande.

6. Un stabilisateur de tube de perfusion selon la revendication 5, dans lequel la cheville (13) est orientée ou profilée de façon à maintenir la bande (9) en position par mise en prise avec le trou (14) à l'intérieur de celle-ci, avant le verrouillage à l'aide du crochet (16).

7. L'invention de la revendication 5 ou de la revendication 6 et dans laquelle le crochet (16), lorsque verrouillé, s'adapte essentiellement au même niveau que, ou encastré sous, les surfaces adjacentes du moyen de verrouillage (12) de façon à essentiellement résister à toute tentative ultérieure de l'ouvrir.
